# EUROPÄISCHE PATENTANMELDUNG

(11) **EP 1 520 883 A1**
(43) Veröffentlichungstag der Anmeldung: **06.04.2005**
(21) Anmeldenummer: 04022277.0
(22) Anmeldetag: 18.09.2004
(51) Int. Cl.: C09C 1/00

(54) **Glänzende schwarze Interferenzpigmente**

(30) Priorität: 01.10.2003 DE 10346167
(71) Anmelder: MERCK PATENT GmbH, 64293 Darmstadt (DE)
(72) Erfinder: Loch, Manuela, 64572 Klein-Gerau (DE); Schupp, Nicole, 64401 Gross-Bieberau (DE); Anselmann, Ralf, Dr., 59348 Luedinghausen-Seppenrade (DE); Heider, Lilia, Dr., Winchester SO22 4HZ (GB)

(57) **Zusammenfassung**

Die vorliegende Erfindung betrifft glänzende schwarze Interferenzpigmente auf der Basis eines Substratgemisches, dadurch gekennzeichnet, dass es
(A) eine Beschichtung aus Fe₃O₄
(B) eine farblose Beschichtung bestehend aus einer oder mehreren Schichten jeweils mit einem Brechungsindex von n ≤ 1,8,
(C) optional eine nicht flächendeckende Belegung mit einem absorbierenden hochbrechenden Material,
   und optional
(D) eine äußere Schutzschicht
aufweist,
sowie deren Verwendung in Farben, Lacken, Pulverlacken, Druckfarben, Kunststoffen, keramischen Materialien, Gläsern, zur Lasermarkierung von Papieren und Kunststoffen, in kosmetischen Formulierungen und zur Herstellung von Pigmentpräparationen und Trockenpräparaten.

## Beschreibung

Die vorliegende Erfindung betrifft glänzende schwarze Interferenzpigmente auf der Basis eines Substratgemisches, das hoch- und niedrigbrechende Schichten aufweist, und deren Verwendung in Farben, Lacken, Pulverlacken, Druckfarben, Kunststoffen, keramischen Materialien, Gläsern, in kosmetischen Formulierungen, zur Lasermarkierung und zur Herstellung von Pigmentpräparationen und Trockenpräparaten.

Glanz- oder Effektpigmente werden in vielen Bereichen der Technik eingesetzt, insbesondere im Bereich der Autolacke, der dekorativen Beschichtung, im Kunststoff, in Farben, Druckfarben sowie in kosmetischen Formulierungen.

Schwarze Pigmente basieren in vielen Fällen auf Kohlenstoff. Derartige Pigmente sind beispielsweise bekannt aus den Offenlegungschriften DE 42 27 082 A1, DE 36 36 076 A1, DE 36 17 430 A1, und EP 0 246 523 B1. Die Herstellung der schwarzen Glanzpigmente erfolgt entweder durch den Einsatz von Ruß, durch Zersetzungsprozesse von organischen Verbindungen oder durch temperaturabhängige Calcinierung von Kohlenwasserstoffen.

Die aus dem Stand der Technik bekannten schwarzen Glanzpigmente haben den Nachteil, dass sie sich durch einen stumpfen Glanz und eine grau-schwarze bzw. braun-schwarze Farbe auszeichnen.

Aus der EP 0 753 545 B1 sind goniochromatische Glanzpigmente auf Basis von mehrfach beschichteten, hochbrechenden, für sichtbares Licht zumindest teilweise durchlässigen, nichtmetallischen, plättchenförmigen Substraten, die mit absorbierenden hochbrechenden Metalloxiden wie Eisen- und Chromoxiden, Ilmenit oder auch Gemischen dieser Oxide belegt sind, bekannt, die mindestens ein Schichtpaket aus einer farblosen niedrigbrechenden und einer reflektierenden, selektiv oder nicht selektiv absorbierenden Beschichtung aufweisen. Die aus dem Stand der Technik bekannten Glanzpigmente zeigen Interferenzfarben, die sehr stark vom Betrachtungswinkel abhängig sind, was in der Mehrzahl der Anwendungen nicht erwünscht ist. Weiterhin sind diese Pigmente zum Teil sehr schwer herstellbar bzw. reproduzierbar.

Aufgabe der vorliegenden Erfindung ist es daher ein anorganisches Pigment zu finden, dass ein glänzendes reines Schwarz aufweist und gleichzeitig sich durch eine hohe Deckkraft und eine gute Verarbeitbarkeit auszeichnet und keine ausgeprägte Goniochromatizität besitzt.

Überraschenderweise wurden nun schwarze Interferenzpigmente mit hohem Glanz auf Basis eines Substratgemisches gefunden, die eine bestimmte Anordnung optisch funktioneller Schichten aufweisen, wodurch besondere Farb- und Glanzeffekte erzielt werden. Auf einem Substratgemisch basierend auf Plättchen unterschiedlicher Partikelgrößen wird zunächst eine definierte Absorption (Absorptionsschicht) erzeugt und anschließend darauf durch Nutzung der Multilayer-Technologie und der Belegung mit mindestens einer niedrigbrechenden semi-transparenten und optional einer absorbierenden Schicht ein Interferenzsystem realisiert. Diese Kombination aus Absorption und Interferenz ergibt ein finales schwarzes Interferenzpigment mit hohem Glanz und einem außergewöhnlich hohen Deckvermögen.

Die erfindungsgemäßen schwarzen Interferenzpigmente zeichnen sich durch einen hohen Schwärzegrad (niedriger L-Wert, a- bzw. b-Werte liegen um den Nullpunkt) und einen intensiven Glanz aus.

Gegenstand der Erfindung sind somit glänzende schwarze Interferenzpigmente auf der Basis eines Substratgemisches, dadurch gekennzeichnet, dass es
(A) eine Beschichtung aus Fe₃O₄,
(B) eine farblose Beschichtung bestehend aus einer oder mehreren Schichten jeweils mit einem Brechungsindex n ≤ 1,8,
(C) optional eine nicht flächendeckende Belegung mit einem absorbierenden hochbrechenden Material,
   und optional
(D) eine äußere Schutzschicht
aufweist.

Gegenstand der Erfindung ist weiterhin die Verwendung der erfindungsgemäßen Pigmente in Farben, Lacken, Pulverlacken, Druckfarben, Kunststoffen, keramischen Materialien, Gläsern und zur Lasermarkierung. Aufgrund der hohen Farbkraft und des guten Hautgefühls sind die erfindungsgemäßen toxisch unbedenklichen Pigmente insbesondere für die dekorative Kosmetik geeignet. Weiterhin können die erfindungsgemäßen Pigmente auch zur Herstellung von Pigmentpräparationen sowie zur Herstellung von Trockenpräparaten, wie z.B. Granulate, Chips, Pellets, Briketts, Würstchen, etc., verwendet werden. Die Trockenpräparate können insbesondere in kosmetischen Formulierungen, Druckfarben und Lacken eingesetzt werden.

Ein geeignetes Basissubstrat für die erfindungsgemäßen schwarzen Pigmente ist ein Gemisch aus groben und feinen transparenten plättchenförmigen Substraten. Bevorzugte Substrate sind Schichtsilikate. Besonders geeignet sind natürlicher und/oder synthetischer Glimmer, Talkum, Kaolin, plättchenförmige Eisen- oder Aluminium-Oxide, Glas-, SiO₂-, TiO₂-, Graphitplättchen, synthetische trägerfreie Plättchen, BiOCl-Plättchen oder andere vergleichbare Materialien.

Die Größe der Basissubstrate ist definiert durch die Zielvorgabe Schwarz und Glanzintensität bzw. Deckkraft und muß dieser jeweils angepasst werden. In der Regel haben die plättchenförmigen Substrate eine Dicke zwischen 0,05 und 5 µm, insbesondere zwischen 0,1 und 4,5 µm. Die Ausdehnung in den beiden anderen Bereichen beträgt üblicherweise 1 bis 200 µm, vorzugsweise 1 bis 150 µm, und insbesondere 1 bis 130 µm.

Bei dem Basissubstrat handelt es sich um Gemische von gleichen oder verschiedenen Substraten jeweils mit unterschiedlichen Partikelgrößen. Das Substratgemisch kann aus zwei, drei oder mehr unterschiedlichen Substraten bestehen. Vorzugsweise besteht das Substratgemisch nur aus einem Substrat mit zwei unterschiedlichen Partikelgrößen. Weiterhin bevorzugt sind Substratgemische bestehend aus synthetischem Glimmer, SiO₂-, TiO₂- oder Glasplättchen.

Die Substrate können in jedem Verhältnis miteinander gemischt werden. In der Regel werden grobe Plättchen mit Partikelgrößen von 10 bis 200 µm, vorzugsweise von 40 bis 200 µm, und insbesondere von 10 bis 130 µm mit feinen Plättchen mit Partikelgrößen von 1 bis 60 µm, vorzugsweise von 5 bis 60 µm, und insbesondere von 10 bis 60 µm, miteinander vor der Belegung gemischt. Für gute Schwärzungseigenschaften des Pigments mit gutem Glanz ist das Verhältnis der feinen und groben Substratpartikel entscheidend. Vorzugsweise werden grobe und feine Substrate im Substratgemisch im Verhältnis 10 : 1 bis 1 : 10, insbesondere im Verhältnis 5 : 1 bis 1 : 5, gemischt. Besonders bevorzugt sind 1 : 1-Gemische. Bevorzugte Substratgemische bestehen aus Glimmerplättchen mit unterschiedlichen Partikelgrößen. Besonders bevorzugte Substratgemische bestehen aus groben und feinen Plättchen, insbesondere aus S-Glimmer (> 125 µm) und F-Glimmer (< 25 µm).

Die Dicke der einzelnen Schichten auf dem Basissubstrat ist wesentlich für die optischen Eigenschaften des Pigments. Insbesondere die Schicht (A) beeinflusst wesentlich die Farbeigenschaften. Die Schicht (B) sollte gegenüber der Schicht (A) vergleichsweise dünn sein. Für ein Pigment mit hoher Farbkraft muß die Dicke der einzelnen Schichten genau aufeinander eingestellt werden.

Die erfindungsgemäßen Interferenzpigmente weisen alternierend eine hochbrechende Schicht (A) bzw. (C) und eine niedrigbrechende Schicht (B) auf. Die hochbrechenden Schichten (A) und (C) besitzen einen Brechungsindex von n > 1,8, vorzugsweise von n ≥ 2,0.

Die Dicke der Fe₃O₄-Schicht (A) beträgt vorzugsweise 1 bis 350 nm, insbesondere 50 nm bis 300 nm und ganz besonders bevorzugt 100 bis 250 nm.

Als farblose niedrigbrechende für die Beschichtung (B) geeignete Materialien sind vorzugsweise Metalloxide bzw. die entsprechenden Oxidhydrate, wie z.B. SiO₂, Al₂O₃, AlO(OH), B₂O₃, ZnSiO₄, MgF₂, MgSiO₃, oder ein Gemisch der genannten Metalloxide, geeignet. Vorzugsweise besteht die Schicht (B) aus SiO₂, MgF₂ oder Al₂O₃ oder deren Gemischen. Die niedrigbrechende Schicht (B) kann aus einer niedrigbrechenden Schicht bestehen oder aus einem Paket von zwei oder mehr niedrigbrechenden Schichten. Vorzugsweise besteht die Schicht (B) aus einer oder zwei niedrigbrechenden Schichten, die von der Zusammensetzung her nicht identisch sind, deren Schichtdicken aber gleich oder verschieden sein können. Vorzugsweise besteht die Schicht (B) aus einer SiO₂-Schicht und/oder einer Al₂O₃-Schicht.

Für die optischen Eigenschaften der erfindungsgemäßen Pigmente ist es von Vorteil, wenn die Schicht (B) relativ dünn ist. Die Dicke der Schicht (B) beträgt unabhängig von der Anzahl der Einzelschichten 1 bis 350 nm, vorzugsweise 50 bis 300 nm und insbesondere 20 bis 250 nm.

Sofern als finale Schicht die absorbierende hochbrechende Schicht (C) aufgebracht wird, so ist diese nur in geringen Konzentrationen von vorzugsweise 2-5 Gew.%, insbesondere 2-3 Gew.%, bezogen auf das eingesetzte Substratgemisch vorhanden und bildet somit keine geschlossene Schicht. Die Schicht (C) besteht vorzugsweise aus Fe₃O₄, ferner aus schwarzen Sulfiden, Oxynitriden, oder deren Mischungen. Die finale Schicht (C) dient der weiteren Optimierung des Glanzes.

Durch die Beschichtung der Substrate mit einer hochbrechenden Schicht (A), einer niedrigbrechenden Schicht (B), und optional einer weiteren schwarzen hochbrechenden Schicht (C) entstehen schwarze Interferenzpigmente, deren Glanz, Deckvermögen in weiten Grenzen variiert werden können.

Besonders bevorzugte Interferenzpigmente weisen folgende Schichtenfolgen auf:
Substratgemisch + Fe₃O₄ (A) + SiO₂ (B)
Substratgemisch + Fe₃O₄ (A) + SiO₂ (B) + Al₂O₃ (B)
Substratgemisch + Fe₃O₄ (A) + SiO₂ (B) + Fe₃O₄ (C)
Substratgemisch + Fe₃O₄ (A) + SiO₂ (B) + Al₂O₃ (B) + Fe₃O₄ (C)
Substratgemisch + Fe₃O₄ (A) + Al₂O₃ (B)
Substratgemisch + Fe₃O₄ (A) + Al₂O₃ (B) + SiO₂ (B)
Substratgemisch + Fe₃O₄ (A) + Al₂O₃ (B) + Fe₃O₄ (C)
Substratgemisch + Fe₃O₄ (A) + Al₂O₃ (B) + SiO₂ (B) + Fe₃O₄ (C)

Durch Kombination der Absorption und der Interferenz auf einem definierbaren Substratgemisch erhält man ein schwarzes glänzendes Interferenzpigment mit einem einstellbaren Farbanteil. Durch die definierte Einstellung der a- und b-Werte ist es möglich, schwarze Interferenzpigmente mit einem Gold-, Grün-, Rot- oder Blaustich zu erhalten.

Die erfindungsgemäßen Interferenzpigmente lassen sich relativ leicht herstellen durch die Erzeugung hoch- und niedrigbrechender Interferenzschichten mit genau definierter Dicke und glatter Oberfläche auf den feinteiligen, plättchenförmigen Substraten.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z.B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Vor der Belegung wird zunächst das Substratgemisch aus groben und feinen Plättchen durch einfaches Mischen hergestellt. Bei der Nassbeschichtung wird das Substratgemisch in Wasser suspendiert und mit einem oder mehreren hydrolysierbaren Metallsalzen bei einem für die Hydrolyse geeigneten pH-Wert versetzt, der so gewählt wird, dass die Metalloxide bzw. Metalloxidhydrate direkt auf den Plättchen ausgefällt werden, ohne daß es zu Nebenfällungen kommt. Der pH-Wert wird üblicherweise durch gleichzeitiges Zudosieren einer Base oder Säure konstant gehalten. Die SiO₂-Schicht wird in der Regel aus Natron- oder Kaliwasserglas hergestellt. Anschließend werden die Pigmente abgetrennt, gewaschen und getrocknet und in einer reduzierenden Atmosphäre vorzugsweise unter Formiergas geglüht, wobei die Glühtemperatur in Hinblick auf die jeweils vorliegende Beschichtung optimiert werden kann. In der Regel liegen die Glühtemperaturen zwischen 500 und 900 °C. Vorzugsweise wird im Formiergasstrom der Zusammensetzung N₂ oder Argon mit einer Konzentration von 4-10 % H₂, insbesondere 4-8 % und ganz besonders bevorzugt mit 8 % H₂ reduziert. Insbesondere besteht das eingesetzte Formiergas aus 92 % N₂ und 8 % H₂. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden. Weiterhin kann die Beschichtung auch in einem Wirbelbettreaktor durch Gasphasenbeschichtung erfolgen, wobei z.B: die in EP 0 045 851 und EP 0 106 235 zur Herstellung von Perlglanzpigmenten vorgeschlagenen Verfahren entsprechend angewendet werden können.

Der Farbton der Pigmente kann in weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder meßtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung (Schicht D) wird die chemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendermedien können funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493.

Es versteht sich von selbst, dass für die verschiedenen Anwendungszwecke die erfindungsgemäßen Interferenzpigmente auch vorteilhaft in Abmischung mit organischen Farbstoffen und/oder Pigmenten, wie z.B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Plättchen auf Basis von Glimmer, Glas, Al₂O₃, Fe₂O₃, SiO₂, etc., verwendet werden können. Die erfindungsgemäßen schwarzen Interferenzpigmente können in jedem Verhältnis mit handelsüblichen Pigmenten und Füllern gemischt werden.

Als Füllstoffe sind z.B. zu nennen natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe. Bezüglich der Partikelform des Füllstoffes gibt es keine Einschränkungen. Sie kann den Anforderungen gemäß z.B. plättchenförmig, sphärisch oder nadelförmig sein.

Die erfindungsgemäßen Pigmente sind aufgrund ihrer guten Hautadhäsion insbesondere geeignet für kosmetische Formulierungen, sowohl im Bereich der dekorativen Kosmetik als auch für Personal Care Produkte. Bei der Verwendung der erfindungsgemäßen Pigmente mit Farbstoffen, Ruß und/oder anderen Effektpigmenten lassen sich mit ihnen besonders wirksame Effekte in den verschiedenen Anwendungsmedien erzielen, z.B. in kosmetischen Formulierungen, wie z.B. Wimperntusche, Eyeliner, Kajalstift.

Selbstverständlich können die erfindungsgemäßen Pigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatine, hochmolekulare Kohlenhydrate und/oder oberflächenaktive Hilfsmittel, etc.

Die die erfindungsgemäßen Pigmente enthaltenden Formulierungen können dem lipophilen, hydrophilen oder hydrophoben Typ angehören. Bei heterogenen Formulierungen mit diskreten wäßrigen und nicht-wässrigen Phasen können die erfindungsgemäßen Pigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen. Den Konzentrationen der erfindungsgemäßen Interferenzpigmente in der Formulierung sind keine Grenzen gesetzt. Sie können - je nach Anwendungsfall - zwischen 0,001 (rinse-off-Produkte, z.B. Duschgele) - 100 % (z.B. Glanzeffekt-Artikel für besondere Anwendungen) liegen. Die erfindungsgemäßen Pigmente können weiterhin auch mit kosmetischen Wirkstoffen kombiniert werden. Geeignete Wirkstoffe sind z.B.

Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erytrolose u.a.) sowie weitere kosmetische Wirkstoffe wie z.B. Bisabolol, LPO, Ectoin, Emblica, Allantoin, Bioflavanoide und deren Derivate.

Die erfindungsgemäßen Pigmente sind weiterhin geeignet zur Herstellung von fließfähigen Pigmentpräparationen und Trockenpräparaten enthaltend ein oder mehrere erfindungsgemäße Pigmente, Bindemittel und optional ein oder mehrere Additive. Unter Trockenpräparaten sind auch Präparate zu verstehen, die 0 bis 8 Gew.%, vorzugsweise 2 bis 8 Gew.%, insbesondere 3 bis 6 Gew.%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0,2-80 mm auf. Die Trockenpräparate finden insbesondere Anwendung bei der Herstellung von Druckfarben und in kosmetischen Formulierungen.

Die erfindungsgemäßen Pigmente sind mit einer Vielzahl von Farbsystemen kompatibel vorzugsweise aus dem Bereich der Lacke, Farben und Druckfarben. Für die Herstellung der Druckfarben ist eine Vielzahl von Bindern, insbesondere wasserlösliche Typen, geeignet, wie sie z.B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen, sowie für Anwendungen im Agrarbereich, z.B. für Gewächshausfolien, sowie z. B. für die Farbgebung von Zeltplanen, geeignet.

Gegenstand der Erfindung ist weiterhin auch die Verwendung der Pigmente in Formulierungen wie Farben, Druckfarben, Sicherheitsdruckfarben, Lacken, Pulverlacken, Kunststoffen, keramischen Materialien, Gläsern, in kosmetischen Formulierungen, als Dotierstoff für die Lasermarkierung von Papieren und Kunststoffen sowie zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1 (Glimmer + Fe₃O₄ + SiO₂ + Al₂O₃)

### Schritt 1.1

42 g S-Glimmer (10-130 µm) und 18 g F-Glimmer (1-15 µm) werden in 1,0 l VE-Wasser suspendiert und unter starkem Rühren auf 80 °C erhitzt. Die Glimmersuspension wird mit 10 %iger Schwefelsäure auf pH = 2,8 eingestellt. Zu dieser Suspension werden 46,05 g Eisen(II)sulfat und 503,49 g Eisen(III)chlorid-Lösung (w_{Fe}) = 15 %) zudosiert, wobei der pH-Wert mit Natronlauge (32 %) konstant gehalten wird. Während der Belegung werden Farbmessungen vorgenommen. Die Dosierung der Eisen(III)chlorid-Lösung wird unterbrochen, sobald der Bunttonwinkel 56° und 59° erreicht ist. Die Suspension wird weitere 60 Minuten bei 80 °C gerührt und der pH-Wert wird mit Natronlauge (w = 32 %) auf pH = 7 eingestellt. Anschließend werden 266,52 g Natronwasserglaslösung (hergestellt aus 133,26 g Natronwasserglas (w_{SiO2}) = 27 % und 133,26 g VE-Wasser) zudosiert, wobei der pH-Wert mit 10%iger Salzsäure konstant auf pH = 7,0 gehalten wird. Nach der Zugabe der Fällungslösung wird weitere 30 Minuten gerührt. Zuletzt werden 96,64 g Aluminiumsulfatlösung (hergestellt aus 24,16 g Aluminiumsulfat-Heptahydrat in 72,48 g VE-Wasser) zudosiert, wobei der pH-Wert mit 32%iger Natronlauge konstant gehalten wird. Die Suspension wird 30 Minuten bei 80 °C gerührt. Zur Aufarbeitung wird das Pigment abfiltriert, mit VE-Wasser gewaschen, bei 110 °C getrocknet und 30 min bei 575 °C unter Formiergasatmosphäre (8 % H₂/92 % N₂) geglüht.

Man erhält intensiv schwarz glänzende Interferenzpigmente, die folgende Farbwerte aufweisen (Minolta Farbmessgerät CR 300):

| | |
|---|---|
| L-Wert | 30 → 32 |
| a-Wert | 0 → 1 |
| b-Wert | -2 → -4 |
| C-Wert (Farbstärke) | 2 → 4 |

### Beispiel 2 (Glimmer + Fe₃O₄ + SiO₂ + Fe₃O₄)

50 g N-Glimmer (10-60 µm) und 50 g F-Glimmer (1-15 µm) werden in 1,2 l VE-Wasser suspendiert und unter starkem Rühren auf 80 °C erhitzt. Die Glimmersuspension wird mit 10 %iger Salzsäure auf pH = 3,2 eingestellt. Zu dieser Suspension werden 153,05 g Eisen(III)chlorid-Lösung (w_{Fe}) = 15 %) verdünnt mit 612,2 g VE-Wasser zudosiert, wobei der pH-Wert mit Natronlauge (32 %) konstant gehalten wird. Während der Belegung werden Farbmessungen vorgenommen. Die Suspension wird weitere 30 Minuten bei 80 °C gerührt und der pH-Wert wird mit Natronlauge (w = 32 %) auf pH = 7,0 eingestellt. Anschließend werden 290 g Natronwasserglaslösung (hergestellt aus 145 g Natronwasserglas (w_{SiO2}) = 27 % und 145 g VE-Wasser) zudosiert, wobei der pH-Wert mit 10 %iger Salzsäure konstant auf pH = 7,0 gehalten wird. Nach der Zugabe der Fällungslösung wird weitere 30 Minuten gerührt. Zuletzt werden 65,40 g Eisen(III)chlorid-Lösung (w_{Fe}) = 15 %) bei pH 3,2 zudosiert, wobei der pH-Wert mit 32 %iger Natronlauge konstant gehalten wird. Die Suspension wird 30 Minuten bei 80 °C gerührt und mit NaOH (32 %) auf pH 7,0 gestellt. Zur Aufarbeitung wird das Pigment abfiltriert, mit VE-Wasser gewaschen, bei 110 °C getrocknet und 30 min bei 575 °C unter Formiergasatmosphäre (8 % H₂/92 % N₂) geglüht.

Man erhält intensiv schwarz glänzende Interferenzpigmente, die folgende Farbwerte aufweisen (Minolta Farbmessgerät CR 300):

| | |
|---|---|
| L-Wert | 36 → 38 |
| a-Wert | -1 → 0 |
| b-Wert | -9 → -6 |
| C-Wert (Farbstärke) | 9 → 6 |

### Anwendungsbeispiele

### Beispiel A: Creme Mascara (O/W)

| | | |
|---|---|---|
| Phase A: | 15 % | Interferenzpigment aus Beispiel 2 |
| | | |
| Phase B: | 8 % | Stearinsäure (1) |
| | 6 % | Bienenwachs (1) |
| | 4 % | Canaubawachs (2) |
| | 3 % | Eutanol (3) |
| | 2 % | Arlacel 83 V (4) |
| | 0,1 % | Propylparaben (1) |
| | 0,5% | Tocopherolacetat (1) |
| | | |
| Phase C: | 50,84 % | Wasser |
| | 2,3 % | Triethanolamin (1 ) |
| | 8 % | Shellac (5) |
| | 0,25 | Methylparaben (1 ) |
| | 0,01 % | Biotin (1) |

### Herstellung:

Alle Bestandteile der Phase B zusammen bei ca. 80 °C aufschmelzen, rühren bis alles geschmolzen ist. Die Interferenzpigmente der Phase A einrühren. Shellac der Phase C im Wasser lösen, auf 75 °C erwärmen. Die restlichen Bestandteile der Phase C zugeben, lösen. Bei 75 °C langsam unter Rühren die Phase C zu Phase A/B geben, 2 min homogenisieren. Die Masse unter Rühren auf Raumtemperatur abkühlen.

### Bezugsquellen:

(1) Merck KGaA
(2) Kahl & Co
(3) Cognis GmbH
(4) Uniqema
(5) Paroxite Ltd.

### Beispiel B: Eyeliner

| | | |
|---|---|---|
| Phase A: | 20 % | Interferenzpigment aus Beispiel 1 |
| | 2 % | Ronasphere® (1) |
| | 0,4 % | Carpobol EDT 2001 (2) |
| | q.s. | Zitronensäure (1) |
| | ad 60 | Wasser |
| Phase B: | 4,0 % | Glycerin, wasserfrei (1) |
| | 0,9 % | Triethanolamin (1) |
| | 2,0 % | Luviskol VA 64 Powder (3) |
| | 1,0 % | Germaben II (4) |
| | 9,7 % | Wasser |

### Herstellung:

Interferenzpigment und Ronasphere® im Wasser der Phase A dispergieren. Mit einigen Tropfen Zitronensäure ansäuern um die Viskosität zu vermindern, Carpobol EDT unter Rühren einstreuen. Nach vollständiger Lösung die vorgelöste Phase B langsam einrühren und pH-Wert auf 7,0-7,5 einstellen.

### Bezugsquellen:

(1) Merck KGaA
(2) Noveon
(3) BASF AG
(4) ISP Global Technologies

## Patentansprüche

1. Glänzende schwarze Interferenzpigmente auf der Basis eines Substratgemisches, **dadurch gekennzeichnet, dass** es
(A) eine Beschichtung aus Fe₃O₄
(B) eine farblose Beschichtung bestehend aus einer oder mehreren Schichten jeweils mit einem Brechungsindex von n ≤ 1,8,
(C) optional eine nicht flächendeckende Belegung mit einem absorbierenden hochbrechenden Material,
und optional
(D) eine äußere Schutzschicht
aufweist.

2. Glänzende schwarze Interferenzpigmente nach Anspruch 1, **dadurch gekennzeichnet, dass** das Substratgemisch aus Plättchen mit unterschiedlichen Partikelgrößen besteht.

3. Glänzende schwarze Interferenzpigmente nach Anspruch 1 oder 2, **dadurch gekennzeichnet, dass** die Plättchen ausgewählt sind aus der Gruppe natürlicher oder synthetischer Glimmer, BiOCI-Plättchen, Glasplättchen, Fe₂O₃-Plättchen, Graphitplättchen, Al₂O₃-Plättchen, SiO₂-Plättchen oder TiO₂-Plättchen.

4. Glänzende schwarze Interferenzpigmente nach einem der Ansprüche 1 bis 3, **dadurch gekennzeichnet, dass** das Substratgemisch aus gleichen oder unterschiedlichen Plättchen besteht, die sich in der Partikelgröße unterscheiden.

5. Glänzende schwarze Interferenzpigmente nach einem der Ansprüche 1 bis 4, **dadurch gekennzeichnet, dass** die Schicht (B) aus SiO₂, Al₂O₃, AIO(OH), B₂O₃, ZnSiO₄, MgF₂, MgSiO₃, oder deren Gemische besteht.

6. Glänzende schwarze Interferenzpigmente nach einem der Ansprüche 1 bis 5, **dadurch gekennzeichnet, dass** die Schicht (A) Schichtdicken von 1 bis 350 nm aufweist.

7. Glänzende schwarze Interferenzpigmente nach einem der Ansprüche 1 bis 6, **dadurch gekennzeichnet, dass** die Schicht (B) Schichtdicken von 10 bis 250 nm aufweist.

8. Glänzende schwarze Interferenzpigmente nach einem der Ansprüche 1 bis 7, **dadurch gekennzeichnet, dass** die Schicht (B) aus einer oder mehreren niedrigbrechenden Schichten besteht.

9. Glänzende schwarze Interferenzpigmente nach einem der Ansprüche 1 bis 8, **dadurch gekennzeichnet, dass** die Schicht (C) aus Fe₃O₄, schwarzen Sulfiden, schwarzen Oxynitriden oder deren Gemische besteht.

10. Glänzende schwarze Interferenzpigmente nach Anspruch 9, **dadurch gekennzeichnet, dass** die Schicht (C) aus Fe₃O₄ besteht.

11. Verfahren zur Herstellung der glänzenden schwarzen Interferenzpigmente nach Anspruch 1, **dadurch gekennzeichnet, dass** die Beschichtung der plättchenförmigen Substrate nasschemisch, durch CVD- oder PVD-Verfahren erfolgt.

12. Verwendung der Interferenzpigmente nach Anspruch 1 in Farben, Lacken, Pulverlacken, Druckfarben, Kunststoffen, keramischen Materialien, Gläsern, in kosmetischen Formulierungen, zur Lasermarkierung und zur Herstellung von Pigmentpräparationen und Trockenpräparaten.

13. Pigmentpräparationen und Trockenpräparate enthaltend ein oder mehrere Interferenzpigmente gemäß Anspruch 1, Bindemittel und optional Additive, Wasser und/oder ein oder mehrere Lösemittel.

14. Trockenpräparate in Form von Pellets, Granulaten, Chips, Briketts und Würstchen enthaltend Interferenzpigmente nach Anspruch 1.
